# EUROPEAN PATENT APPLICATION

(11) **EP 0 624 347 A1**
(43) Date of publication of application: **17.11.1994**
(21) Application number: 94303287.0
(22) Date of filing: 06.05.1994
(51) Int. Cl.: A61B 17/36, A61B 17/42

(54) **Cryoprobe**

(30) Priority: 10.05.1993 GB 9309598
(71) Applicant: BROWNING HEALTHCARE LIMITED, Bristol, BS6 6TL (GB)
(72) Inventor: Browning, James Johnathon, Bristol BS6 6TL (GB)
(74) Representative: Dean, John Paul

(57) **Abstract**

There is provided a cryoprobe for ablating a body surface, the cryoprobe comprising one or more non-linear elements adapted to receive refrigerant medium and arranged to conform to the body surface, such as the surface of a T-shaped body cavity, e.g. the uterus.

One or more such elements may be kinked, curved or bent so that the probe is Y- or T-shaped in use.

The elements may be flexible or articulated, and urged into the use configuration by a resiliant member.

## Description

This invention relates to a cryoprobe and is particularly, though not exclusively, concerned with an endometrial cryoprobe.

The occurrence of both prolonged and heavy periods is a major problem for some women in their 30's, 40's and early 50's. Current methods of treatment for this condition have certain disadvantages as discussed below.

The most radical approach to this problem, a hysterectomy, is disadvantageous in that it is a major operation which must be carried out under a general anaesthetic. The operation is also costly to perform.

Currently available medications work poorly or have unacceptable side effects.

Hysteroscopic transcervical resection of the endometrium (surgery on the endometrial wall performed through the cervix with the aid of an optical fibre probe) is an in-patient procedure requiring a general anaesthetic which is also costly.

Radio frequency endometrial ablation (RFEA) has been used on an out-patient basis (e.g Phipps, J.H et al. Lancet 1990 335:374-376). However, the equipment required is relatively costly - a typical unit costs about £60,000 - and the treatment does not satisfactorily ablate the fundal uterine wall.

Furthermore, dilation of the cervix to about 10 mm is required which is unsatisfactory.

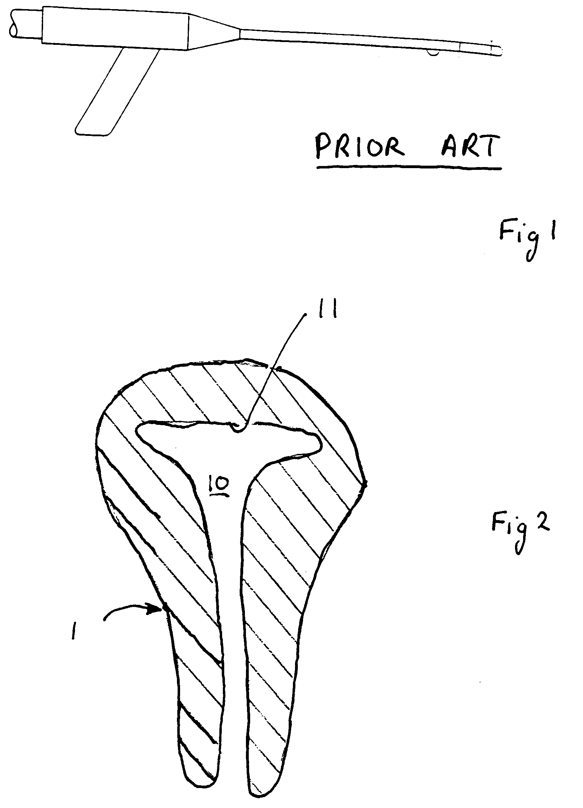

Endometrial cryosurgery employing a straight urological cryoprobe (Spembly DFS - 30 range) shown in elevation in the accompanying drawing Figure 1 and using liquid nitrogen as the refrigerant has been reported (Davies, W.A.R et al J. Obstetrics and Gynaecology (1985) 6, 117-119). However, the cryoprobe used may be disadvantageous in that it does not satisfactorily ablate the fundal endometrial wall because its shape does not conform to the complex configuration of the uterine cavity.

It is an object of the present invention to provide an improved apparatus and method for endometrial ablation.

According to one aspect of the invention there is provided a cryoprobe for ablating a human or animal body surface, preferably a surface of a substantially T-shaped body cavity, the cryoprobe comprising one or more elements adapted to receive refrigerant medium and which extend in use into the arms of the cavity. Preferably, the cavity is that of the human uterus.

Where the probe comprises one element, that element may be curved kinked or bent in plan view.

Where the probe comprises two elements, each element may be curved or kinked or bent, whereby the probe is substantially Y-shaped in use, or L-shaped in use so that the probe is substantially T-shaped in use.

The elements may be formed from a metal, plastics or ceramic material. Preferably plastics include polyolefins, fluorocarbon polymers, polyurethane, and polyacetates. Preferably, the elements are formed from a copper or other metal alloy.

The elements may be retained within a collar or sleeve or other cover during insertion into the cavity to avoid damage to the entrance to the cavity. The collar sleeve or other cover is preferably slidable to expose leading portions of the elements.

Where the cryoprobe includes only one element a proximal portion of the cryoprobe may be insulated to prevent damage to the entrance to the cavity.

Where the cryoprobe is T-shaped in use, at least one of the elements may be urged into an operative in use condition by one or more resilient members. Preferably, both elements are so urged.

According to another aspect of the invention there is provided a method of ablating a lining of a human or animal body surface comprising placing a cryoprobe in accordance with the invention adjacent or in contact with the body surface and passing refrigerant medium into the cryoprobe.

Preferably, the body surface is that of a body cavity, preferably the uterine cavity and the cryoprobe is inserted into the cavity before the refrigerant medium is passed in to the cryoprobe.
Cryoprobes, and a method for their use, in accordance with the invention will now be described, by way of example only, with reference to the further accompanying drawings, Figures 2 to 7 in which;
Fig. 2 is a schematic coronal section through a uterus;
Fig. 3 is a partial longitudinal section through a probe in accordance with one embodiment of the invention in a retracted condition;
Fig. 4 is another partial longitudinal cross-section of the probe of Fig. 3 but in an extended condition;
Fig. 5 is a partial longitudinal cross-section through a probe in accordance with another embodiment of the invention in a retracted condition;
Fig. 6 is another partial longitudinal cross-section through the probe of Fig. 5 but in an extracted condition; and
Fig. 7 is a plan view of a probe in accordance with a further embodiment of the invention.

The uterus 1 has a substantially T shaped cavity 10, which is typically 7 to 9 cm long in the adult female, as shown in Fig. 2. A fundal wall 11 forms the distal surface of the uterus.

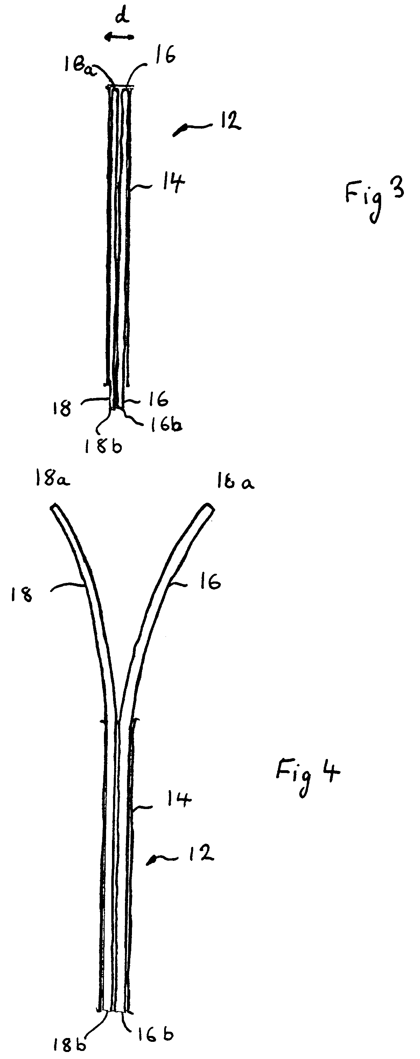

The probe 12 shown in Figs.3 and 4 comprises an elongate collar 14 made of an inert plastics material such as polytetrafluoroethylene (PTFE) which surrounds the ends of two blind-bored elements 16, 18. The elements 16, 18 are made of a metal which is resilient at room and low temperatures, typically a copper alloy, and are formed to have the arcuate shape shown in Fig. 4. The elements 16, 18 have blind ends 16a, 18a respectively. The open ends 16b, 18b of the elements 16, 18 respectively are adapted to be connected to a supply of a refrigerant medium such as nitrous oxide. The diameter d of the collar or sleeve 14 is about 5 mm.

In use, the probe is inserted into the uterine cavity through the cervix under local anaesthetic in the condition shown in Fig. 3. Insertion of the probe is monitored using ultrasound techniques. The relatively low diameter of the collar facilitates insertion of the probe and improves patient comfort. After insertion, the collar 14 is retracted allowing the elements 16, 18 to spring apart into the Y-shaped condition shown in Fig. 4 whereby the elements extend into the arms of the uterine cavity i.e towards the fallopian tubes.

The open ends of elements 16, 18 are then connected to the liquid nitrous oxide supply and liquid NO₂ is passed into the probe in accordance with conventional practice. The rapid cooling of the probe (to about -140°C) due to the liquid NO₂ causes the formation of an ice ball about the probe which ablates the entire endometrial lining to a depth of about 5 mm. The shape of the probe ensures that the fundal wall is sufficiently ablated thus overcoming a disadvantage of prior apparatus and techniques. After thawing, the collar 14 may then be returned to the position shown in Fig. 3 prior to removal through the cervix.

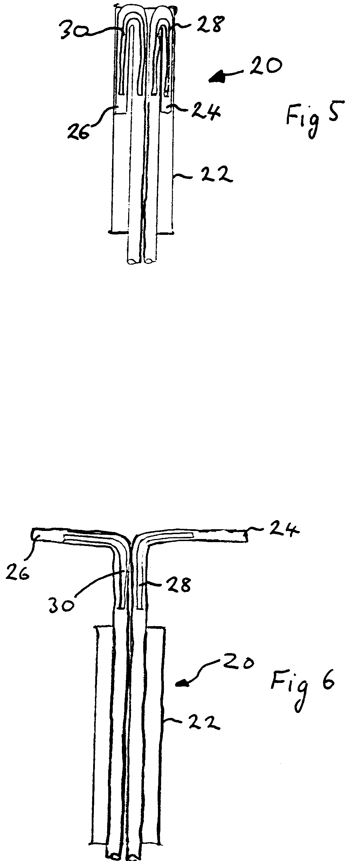

The probe 20 shown in Figs. 5 and 6 comprises a retractable collar 22, which is similar to the collar 14 of the probe 12 described above, and which surrounds the ends of two blind bored flexible elements 24, 26 in a substantially linear non-use configuration shown in Fig. 5. The elements 24, 26 have resilient members 28, 30 embedded into their respective outer surfaces.

In use the probe 20 is inserted into the endometrial cavity as described for probe 12 above. Retraction of the collar 22 causes the resilient members 28, 30 to urge the flexible elements 24, 26 into the position shown in Fig. 6 wherein the probe adopts a substantially T-shaped in use configuration. Nitrous oxide is then passed into the probe as described above. The resilient members 28, 30 are sufficiently flexible to allow the probe to be removed from the uterus with the collar 26 in the position shown in Fig. 6, the ends of the elements 24, 26 being deflected by the uterus and cervix.

The probe 20 may be advantageous over the probe 12 in allowing better contact with the fundal wall.

The cryoprobes 12, 20 have a relatively large (about 25 to 30cm) uninsulated length compared to conventional cryoprobes in which only the final 4 to 5 cm is uninsulated. The increased surface are available for ice formation in the cryoprobes of the present invention is advantageous over conventional designs.

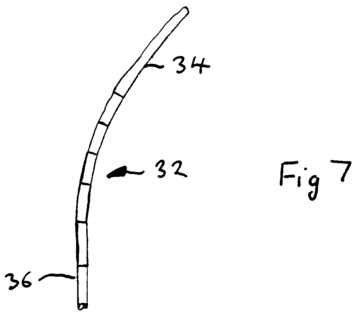

The cryoprobe 32 shown in Fig. 7 comprises a curved blind bored element 34. The element 34 is made of copper alloy and is shaped to extend into an arm of the T-shaped endometrial cavity in use.

The lower portion 36 of the element is relatively insulated to minimize damage to the cervix and lower uterine cavity in use.

In use, the probe is inserted as described above for probes 12 and 20, under ultrasound monitoring, until the blind end of the element 34 extends into one arm of the uterus. Refrigerant medium is passed into the element as described above and after freezing has occurred, the ice ball is thawed and the probe is removed. The probe is then reinserted in the opposite orientation wherein it extends into the opposite arm of the uterus. Cryodestruction is then performed again.

This probe is advantageous in that it is of a relatively simple construction and is therefore durable.

## Claims

1. A cryoprobe for ablating a body surface, the cryoprobe comprising one or more non-linear elements adapted to receive refrigerant medium and arranged to conform to the body surface.

2. A cryoprobe according to claim 1 for ablating a body surface of a substantially T-shaped body cavity having two minor arms, the cryoprobe comprising one or more elements adapted to receive a refrigerant medium and which extend(s) in use into the arms of the cavity.

3. A cryoprobe according to claim 1 or 2 which is an endometrial cryoprobe.

4. A cryoprobe according to any preceding claim and which comprises one refrigerant receiving element in which the said element is curved or kinked in plan view.

5. A cryoprobe according to any one of claims 1 to 3 in which each refrigerant receiving element is curved, kinked or bent whereby the probe is substantially Y-shaped in use; or L-shaped in use so that the cryoprobe is substantially T-shaped in use.

6. A cryoprobe according to claim 5 in which at least one refrigerant receiving element is flexible or articulated whereby it can move from a substantially straight configuration to a substantially L-shaped configuration.

7. A cryoprobe according to claim 6 in which the or each flexible or articulated refrigerant receiving element is urged into the in use configuration by a resilient member.

8. A cryoprobe according to claim 7 which is substantially T-shaped in use and in which both articulated refrigerant receiving elements are urged into position by resilient members.

9. A cryoprobe according to any preceding claim in which the or each refrigerant receiving element is retained within a collar, sleeve or other cover prior to use.

10. A cryoprobe according to claim 9 in which the or each refrigerant receiving element is held in a substantially linear non-use configuration within the collar, sleeve or other cover.
